# EUROPÄISCHE PATENTSCHRIFT

(11) **EP 2 020 601 B1**
(45) Veröffentlichungstag und Bekanntmachung des Hinweises auf die Patenterteilung: **17.11.2010**
(21) Anmeldenummer: 07015258.2
(22) Anmeldetag: 03.08.2007
(51) Int. Cl.: G01N 33/543, G01N 33/58, G01N 21/76

(54) **Kit und Vorrichtung zum Erzeugen einer Chemolumineszenzstrahlung**
Kit and device for creating chemo luminescent radiation
Kit et dispositif destinés à la production d'un rayonnement chimioluminescent

(43) Veröffentlichungstag der Anmeldung: 04.02.2009
(73) Patentinhaber: Micronas GmbH, 79108 Freiburg i. Br. (DE)
(72) Erfinder: Klapproth, Holger, Dr., 79108 Freiburg (DE); Mohry, Sonja, 79108 Freiburg (DE)
(74) Vertreter: Huwer, Andreas

(56) Entgegenhaltungen:
- EP-A- 0 235 970
- EP-A- 0 236 997
- WO-A-2006/040142
- DE-A1- 10 245 435
- US-A- 4 859 583
- US-A- 5 942 407

## Beschreibung

Die Erfindung betrifft einen Kit nach dem Oberbegriff von Anspruch 1 und eine Vorrichtung nach dem Oberbegriff von Anspruch 4.

Aus DE 10 2004 050 032 A1 ist eine Vorrichtung bekannt, die eine Messkammer aufweist, in deren Innenhöhlung an mehreren matrixförmig angeordneten Teststellen jeweils Rezeptoren immobilisiert sind. Die Rezeptoren sind für Liganden bindungsspezifisch, von denen vermutet wird, dass sie in einer zu untersuchenden Probe enthalten sind. Zur Untersuchung der Probe wird diese derart mit den Rezeptoren in Kontakt gebraucht, dass die Liganden an die Rezeptoren binden können. Anschließend werden eventuell noch vorhandene freie Liganden aus der Messkammer entfernt. Die an einen Rezeptor gebundenen Liganden werden mit biotinylierten Nachweisantikörpern in Kontakt gebracht, die an die Liganden binden. Dann werden die Nachweisantikörper mit einem Streptavidin-Peroxidase-Enzym-Marker markiert und eventuell noch vorhandene freie Streptavidin-Peroxidase-Enzym-Marker werden aus der Messkammer entfernt. Danach werden die Teststellen mit Wasserstoffperoxid und Luminol in Kontakt gebracht. Bei Anwesenheit des Peroxidase-Enzyms zersetzt sich das Luminol, wobei eine Chemolumineszenzstrohlung freigesetzt wird. Diese wird mit Hilfe von Sensoren detektiert um die Liganden nachzuweisen und/oder deren Konzentration in der Probe zu bestimmen. Die Vorrichtung hat den Nachteil, dass das Wasserstoffperoxid instabil ist und gekühlt gelagert werden muss. Auch das Peroxidase-Enzym ist sehr empfindlich und wird bei -20 °C gelagert. Die Handhabung der Vorrichtung und der benötigten Reagenzien ist deshalb in der Praxis noch ziemlich umständlich. Auch ist die Vorrichtung für eine dezentrale Verwendung nur bedingt geeignet, wenn am Ort der Verwendung keine Kühleinrichtung vorhanden ist.

Aus DE 102 45 435 A1 ist außerdem eine Vorrichtung zur Detektion von in einer zu untersuchenden Probe enthaltenen Liganden bekannt, die eine Messkammer aufweist, in der an mehreren Teststellen jeweils Rezeptoren immobilisiert sind. Der Nachweis der Bindung der Liganden an die Rezeptoren erfolgt mit Hilfe von Nachwelsantikörpern, die mit einem optischen Marker markiert sind und in lyophilisierter Form in der Messkammer deponiert sind. Beim Befüllen der Messkammer mit der Probe lösen sich die lyophilisierten Nachweisantikörper-Marker-Komplexe und binden an die Liganden. Durch Bestrahlung mit einer Anregungsstrahlung werden die Marker zur Aussendung einer Lumineszenzstrahlung angeregt Diese wird mit Hilfe von optischen Sensoren detektiert. Die Vorrichtung kann zwar über einen längeren Zeitraum bei Raumtemperatur gelagert werden, ohne dass die Nachweisantikörper-Marker-Komplexe nennenswert geschädigt werden. Dennoch ist die Handhabung der Vorrichtung noch relativ aufwändig, weil zur Erzeugung der Anregungsstrahlung für die Marker eine Lichtquelle benötigt wird.

Aus US-A-5 942 407 ist ferner eine gattungsfremde Trocken-Vorrichtung zur Detektion von Analyten in einer Probe bekannt. Die Vorrichtung weist einen Teststreifen mit drei Zonen auf In einer erste Zone sind enzymkonjugierte Liganden, in einer zweiten Zone immobilisierte Analyten und in einer dritten Zone ist ein Reportersystem zur Aktivierung einer Lichtgenerierung mittels eines Enzymkonjugats angeordnet. Die Analyt-Enzym-konjugierten Ligand-Komplexe werden nicht in der zweiten Zone gefangen, sondern wandern weiter in die dritte Zone, wo eine Reaktion zwischen dem Enzym und einer in der dritten Zone vorhandenen Aktivierungssubstanz stattfindet und zur Lichtemission führt. In einer Ausführungsform weist das System Harnstoff-Wasserstoffperoxid, Luminol und einen HRP-konjugierten Lganden auf Hamstotf-Wasserstoffperoxid setzt bei Kontakt mit Wasser Wasserstoffperoxid frei. Dies führt zur Aktivierung des HRP-Enzyms und zur Erzeugung von Licht durch die Reaktion von Peroxidase und Luminol, welches in der dritten Zone vorhanden ist. Die Anwesenheit des Harnstoffs stört jedoch die Bindung zwischen Rezeptor und Ligand.

Aus US-A-4 859 583 ist ferner eine Vorrichtung zur Erzeugung eine Chemilumineszenzstrahlung in Abhängigkeit von der Bindung eines Liganden an einen Rezeptor oder in Abhängigkeit von der Bindung von Antigenen an Antikörper bekannt. Die benötigten Reagenzien sind in lyophilisierter Form in der Vorrichtung vorhanden. Durch die Auftragung der Probe auf die Vorrichtung werden die Reagenzien gelöst. Dabei wird Wasserstoffperoxid von Glucose-Oxidase produziert und wandert zu einem Peroxidase-Marker, an dem Luminol anwesend ist. Das freigestezte Wasserstoffperoxid reagiert chemisch mit dem Peroxidase-konjugierten Liganden und dem Luminol, wobei eine Lumineszenzstrahlung chemisch erzeugt wird.

Es besteht deshalb die Aufgabe, einen Kit und eine Vorrichtung der eingangs genannten Art zu schaffen, die eine einfache Handhabung ermöglicht.

Diese Aufgabe wird bezüglich des Kits mit den Merkmalen des Anspruchs 1 und bezüglich der bezüglich der Vorrichtung mit den Merkmalen des Anspruchs 4 gelöst.

In vorteilhafter Weise kann der Kit bzw. die Vorrichtung dann bei Raumtemperatur über einen längeren Zeitraum aufbewahrt werden, ohne dass sich die Eigenschaften der für die Messung benötigten Substanzen wesentlich verändern. Eine komplizierte Kühlung der Substanzen entfällt also. Der Kit ist deshalb insbesondere auch für eine dezentrale Verwendung geeignet, beispielsweise bei einem Endverbraucher. Sobald die Festsubstanz mit Wasser in Kontakt gerät, wird das für die Erzeugung der Chemolumineszenzstrahlung benötigte Wasserstoffperoxid freigestzt. Da die Lumineszenzstrahlung chemisch erzeugt wird, wird für die Messung keine Anregungslichtquelle benötigt Der Kit ist also leicht handhabbar. Das Ammoniumpersulfat ((NH₄)₂S₂O₈) kann in fester Form bei Raumtemperatur aufbewahrt werden.

Zweckmäßigervveise weist der Marker mindestens einen Streptavidin-Peroxidase-Enzym-Komplex auf. Das Stretdavidin kann dann - wenn es in der Probe gelöst ist - an einen für den Liganden bindungsspezifischen Nachweisantikörper binden, um dadurch den Liganden indirekt mit dem Peroxidase-Enzym zu markieren.

Vorteilhaft ist, wenn der Kit wenigstens einen für den Liganden bindungsspezifischen Nachweisantikörper oder ein funktionelles Fragment eines solchen in lyophilisierter Form aufweist. Bei der erfindungsgemößen Vorrichtung kann ein solcher lyophilisierter Nachweisantikörper oder ein funktionelles Fragment eines solchen in dem mindestens einen zweiten Kompartiment angeordnet sein. Der Nachweisantikörper kann derart ausgestaltet sein, dass er bei einem Kontakt mit dem Peroxidase-Enzym direkt oder indirekt an dieses bindet. Es ist aber auch möglich, dass der Nachweisantikörper bereits an das Peroxidase-Enzym gebunden und der entsprechende Nachweisantikörper-Peroxidase-Enzym-Komplex in lyophilisierter Form vorliegt.

Bei einer bevorzugten Ausgestaltung der Erfindung weist die Innenhöhlung ein drittes Kompartiment auf, in dem der wenigstens eine Rezeptor immobilisiert ist, wobei das erste und das zweite Kompartiment jeweils eine Einlassöffnung und jeweils eine mit dem dritten Kompartiment verbundene Auslassöffnung aufweisen. Dadurch kann während der Lagerung der Vorrichtung ein Kontakt der in dem ersten und zweiten Kompartiment befindlichen Substanzen mit dem in dem dritten Kompartiment angeordneten wenigstens einen Rezeptor vermieden werden.

Vorzugsweise weist das dritte Kompartiment einen Fluidauslass auf, wobei der wenigstens eine Rezeptor zwischen dem ersten und/oder zweiten Kompartiment einerseits und dem Fluidauslass andererseits angeordnet ist. Mit dem Fluidauslass kann gegebenenfalls ein saugfähiges Medium verbunden sein, das die aus der Probe und den im ersten und zweiten Kompartimenten gespeicherten Substanzen bestehende Lösung nach dem Inkontaktbringen mit dem wenigstens einen Rezeptor aufnimmt.

Erwähnt werden soll noch, dass das mit dem Salz stabilisierte Luminol und die Festsubstanz auch in getrennten Kompartimenten angeordnet sein können.

Nachfolgend ist ein Ausführungsbeispiel der Erfindung anhand der Zeichnung näher erläutert. Es zeigt
- Fig. 1: einen Längsschnitt durch eine Vorrichtung zum Erzeugen einer Chemo- lumineszenzstrahlung,

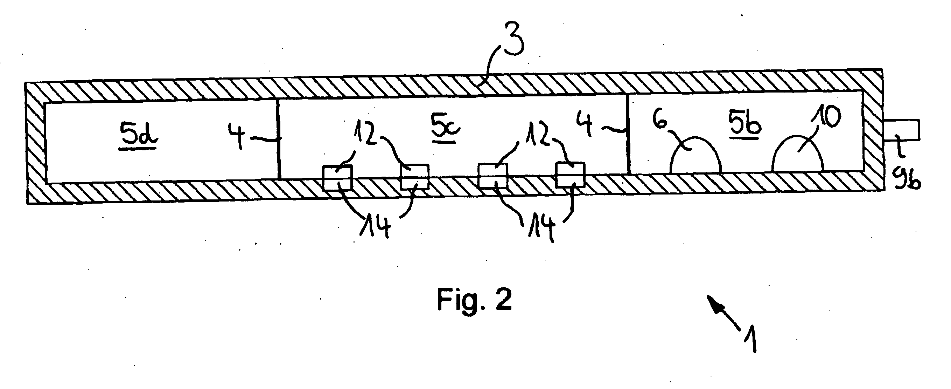

- Fig. 2: einen Querschnitt durch die Vorrichtung entlang der In Fig. 1 mit II bezeichneten Ebene, und
- Fig. 3: eine über einen Nachwelsantikörper an einen Rezeptor gebundenen und mit einem optischen Marker markierten Liganden.

Eine Vorrichtung 1 zum Nachweise und/oder Bestimmen der Konzentration von Liganden 2 in einer Wasser enthaltenden, flüssigen oder fileßfählgen Probe hat eine Russzelle 3 mit einer Innenhöhlung, die durch Trennwände 4 in mehrere Kompartimente 5q, 5b, 5c, 5d unterteilt Ist.

In einem ersten Kompartiment 5a ist ein Feststoff 7 deponiert, der als optische Marker 8 Streptavidin-HRP-Komplexe in lyophilisierter Form enthält Zur Stabilisierung der Marker 8 enthält der Feststoff 7 einen nicht reduzierenden Zucker, ein Protein der LEA-Kiasse und ein Antioxidanzmittel.

Das erste Kompartiment 5a hat eine erste Einlassöffnung 9a, durch welche die Probe beispielsweise mittels einer Pipette In das erste Kompartiment 5a eingefüllt werden kann. Bei einem Kontakt mit der Probe löst sich der Feststoff 7 In der Probe.

In einem zweiten Kompartiment 5b sind mit einem Salz stabilisiertes Luminol 6 und eine Festsubstanz 10 vorzugsweise mit Abstand zueinander deponiert. Die Festsubstanz 10 enthält einen Träger, insbesondere Harnstoff, an den Wasserstoffperoxid gebunden Ist Das zweite Kompartiment 5b hat eine zweite Einlassöffnung 9b, durch welche die Probe und/oder eine wässrige Flüssigkeit In das zweite Kompartiment 5b eingefüllt werden kann.

Bei einem Kontakt mit der Probe und/oder der wässrigen Flüssigkeit lösen sich das Luminol und der Harnstoff in dieser, wobei das Wasserstoffperoxid freigesetzt wird.

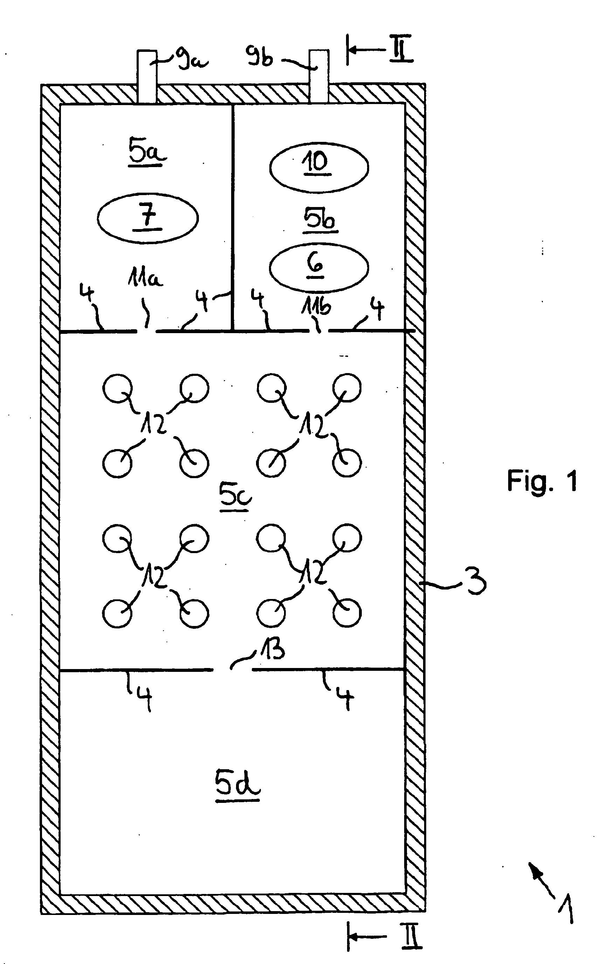

Das erste Kompartiment 5a und das zweite Kompartiment 5b haben jeweils eine Auslassöffnung 11a, 11b, die mit einem dritten Kompartiment 5c verbunden ist An den Auslossöfinungen 11a, 11b können Ventile angeordnet sein, die einen Rückfluss einer in dem dritten Kompartiment 5c befindlichen Flüssigkeit in das erste Kompartiment 5a und/oder zweite Kompartiment 5b verhindern. Wie In Fig. 1 besonders gut erkennbar ist, ist der Feststoff 7 zwischen der ersten Einlassöffnung 9a und der Auslassöffnung 11a deponiert. In entsprechender Weise sind das Luminol 6 und die Festsubstanz 10 zwischen der zweiten Einlassöffnung 9b und der Auslassöffung 11b angeordnet.

In einem dritten Kompartiment 5c sind mehrere Teststellen matrixförmig an einer Begrenzungswand der Innenhöhlung vorgesehen. An jeder Teststelle ist jeweils mindestens ein Rezeptor 12 immobilisiert, der für einen Liganden 2 bindungsspezifisch ist, von dem vermutet wird, dass er in der Probe enthalten Ist. Unter jeder Teststelle ist jeweils ein optischer Sensor 14 in die Begrenzungswand der Flusszelle 3 Integriert

Das dritte Kompartiment 5c hat einen Fluidauslass 13, der mit einem vierten Kompartiment 5d verbunden ist Im vierten Kompartiment 5d ist ein in der Zeichnung nicht näher dargestelltes saugfähiges Medium angeordnet. Die Rezeptoren 12 sind zwischen den Auslassöffnungen 11a, 11 b und dem Fluidauslass 13 angeordnet.

Zum Untersuchen der Probe wird diese zunächst durch die erste Einlassöffnung 9a In das erste Kompartiment 5a eingefüllt. Dabei löst sich der Feststoff 7 In der Probe und die Lösung gelangt durch die Auslassöffnung 11a hindurch in das dritte Kompartiment 5c

In dem dritten Kompartiment 5c gelangt die Probe derart mit den Rezeptoren 12 In Kontakt, dass die liganden 2 - sofern sie In der Probe enthalten sein sollten - an die Rezeptoren 12 binden können In der Probe sind Nachweisanffkörper 15 enthalten, die an die Liganden 2 gebunden sind und/oder an diese binden.

Nun wird eine Spülflüssigkeit über die erste Einlassöffnung 9a und die Auslassöffnung 11a in das dritte Kompartiment 5c eingeleitet, die eventuell noch vorhandene freie Liganden 2 und/oder Nachwelsantikörper 15 aus dem dritten Kompartiment 5c entfernt und in das vierte Kompartiment 5d abtransportiert.

Dann wird die Probe oder die bereits erwähnte wässrige Flüssigkeit durch die zweite Einlassöffnung 9b in das zweite Kompartiment 5b eingefüllt, um das Luminol 6 und die Festssubstanz 10 zu lösen. Dabei wird Wasserstoff-Peroxid freigesetzt und gelangt zusammen mit dem Luminol 6 durch die Auslassöffnung 11 b hindurch in das dritte Kompartiment 5c.

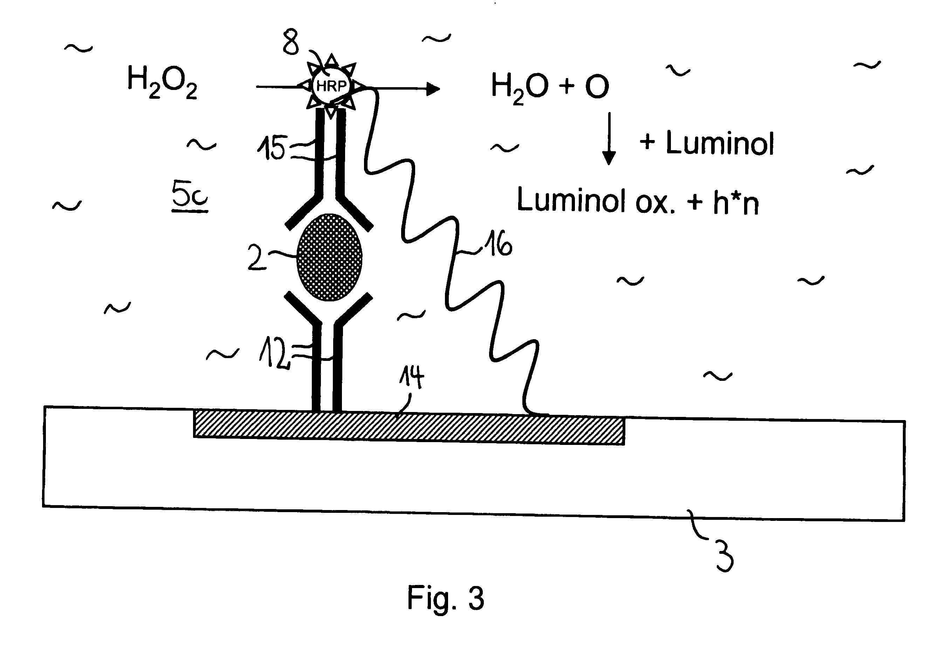

Wenn das Wasserstoffperoxid und das Luminol mit einem an einen Rezeptor 12 gebundenen Streptavidin-HRP-Komplex in Kontakt gerät, wird das Luminol unter Freisetzung von Chemolumineszenzstrahlung 16 zersetzt (Fig. 3). Die Chemolumineszenzstrahlung 16 wird an den einzelnen Teststellen jeweils mit Hilfe des Sensors 14 detektiert. In Abhängigkeit von den so erhaltenen Messsignalen wird die Konzentration des betreffenden Liganden 2 In der Probe ermittelt.

## Patentansprüche

1. Kit zum Erzeugen einer Chemolumineszenzstrahlung (16) in Abhängigkeit von der Bindung mindestens eines in einer zu untersuchenden Probe enthaltenden Liganden (2) an wenigstens einen für den Liganden (2) bindungsspezifischen Rezeptor (12), mit einem Blochip, der einen Träger aufweist, auf dem der wenigstens eine Rezeptor (12) immobilisiert ist, mit mindestens einem wenigstens ein Peroxidase-Enzym aufweisenden Marker (8) zum Markieren des Liganden (2) und mit Luminol (6) das mit einem Salz stabilisiert ist, **dadurch gekennzeichnet, dass** der Kit eine Ammoniumpersulfat enthaltende Festsubstanz (10) aufweist, die bei einem Kontakt mit Wasser Wasserstoffperoxid freisetzt, und dass der Kit einen Natrium-Ascorbat enthaltenden Feststoff (7) umfasst, der den Marker (8) in lyophilisierter Form enthält.

2. Kit nach Anspruch 1, **dadurch gekennzeichnet, dass** der Marker (8) mindestens einen Streptavidin-Peroxidase-Enzym-Komplex aufweist

3. Kit nach Anspruch 1 oder 2, **dadurch gekennzeichnet, dass** er wenigstens einen für den Liganden (2) bindungsspezifischen Nachweisantikörper (15) oder ein funktionelles Fragment eines solchen in lyophilisierter Form enthält, und dass der Nachweisantikörper (15) an das Peroxidase-Enzym gebunden ist und/oder bei einem Kontakt mit dem Peroxidase-Enzym direkt oder indirekt an dieses bindet.

4. Vorrichtung (1) zum Erzeugen einer Chemolumineszenzstrahlung (16) in Abhängigkeit von der Bindung mindestens eines in einer zu untersuchenden Probe enthaltenden Liganden (2) an wenigstens einen für den Liganden (2) bindungsspezifischen Rezeptor (12), mit einer eine Innenhöhlung aufweisenden Flusszelle (3), wobei die Innenhöhlung eine Begrenzungswand hat, auf welcher der wenigstens eine Rezeptor (12) immobilisiert ist, mit mindestens einem wenigstens ein Peroxidase-Enzym aufweisenden Marker (8) zum Markieren des Liganden (2) und mit Luminol (6), das mit zumindest einem Salz stabilisiert ist, **dadurch gekennzeichnet, dass** die Innenhöhlung mindestens zwei Kompartimente (5a, 5b, 5c, 5d) aufweist, dass in mindestens einem ersten Kompartiment (5a) ein Natrium-Ascorbat enthaltender Feststoff (7) deponiert ist, der den Marker (8) in lyophilisierter Form enthält, und dass in mindestens einem zweiten Kompartiment (5b) das mit dem Salz stabilisierte Luminol und eine Ammoniumpersulfat aufweisende Festsubstanz (10) enthalten sind, die bei einem Kontakt mit Wasser Wasserstoffperoxid freisetzt.

5. Vorrichtung (1) nach Anspruch 4, **dadurch gekennzeichnet, dass** der Marker (8) mindestens einen Streptavidin-Peroxidase-Enzym-Komplex aufweist

6. Vorrichtung (1) nach Anspruch 4 oder 5, **dadurch gekennzeichnet, dass** das mindestens eine erste Kompartiment (5a) wenigstens einen für den Liganden (2) bindungsspezifischen Nachweisantikörper (15) oder ein funktionelles Fragment eines solchen in lyophilisierter Form enthält.

7. Vorrichtung (1) nach einem der Ansprüche 5 bis 6, **dadurch gekennzeichnet, dass** die Innenhöhlung ein drittes Kompartiment (5c) aufweist, in dem der wenigstens eine Rezeptor (12) immobilisiert ist, und dass das erste Kompartiment (5a) und das zweite Kompartiment (5b) jeweils eine Einlassöffnung (9a, 9b) und jeweils eine mit dem dritten Kompartiment (5c) verbundene Auslassöffnung (11a, 11b) aufweisen.

8. Vorrichtung (1) nach einem der Ansprüche 4 bis 7, **dadurch gekennzeichnet, dass** das dritte Kompartiment (5c) einen Fluidauslass (13) aufweist, und dass der wenigstens eine Rezeptor (12) zwischen dem ersten Kompartiment (5a) und/oder zweiten Kompartiment (5b) einerseits und dem Fluidauslass (13) andererseits angeordnet ist.

## Claims

1. Kit for generating chemiluminescence (16) as a function of the binding of at least one ligand (2) present in a sample to be tested to at least one receptor (12) which specifically binds the ligand (2), having a biochip which features a support on which the at least one receptor (12) is immobilized, having at least one label (8), featuring at least one peroxidase, for labelling the ligand (2), and having luminol (6) which is stabilized with a salt, **characterized in that** the kit features an ammonium-persulphate-comprising solid substance (10) which releases hydrogen peroxide upon contact with water, and that the kit comprises a sodium-ascorbate-comprising solid (7) in which the label (8) is present in lyophilized form.

2. Kit according to Claim 1, **characterized in that** the label (8) features at least one streptavidin-peroxidase complex.

3. Kit according to either Claim 1 or 2, **characterized in that** it comprises, in lyophilized form, at least one detection antibody (15) which specifically binds the ligand (2) or one functional fragment of such an antibody, and that the detection antibody (15) is bonded to the peroxidase and/or binds directly or indirectly to the peroxidase upon contact with it.

4. Apparatus (1) for generating chemiluminescence (16) as a function of the binding of at least one ligand (2) present in a sample to be tested to at least one receptor (12) which specifically binds the ligand (2), having a flow cell (3) featuring an inner cavity, wherein the inner cavity has a boundary wall on which the at least one receptor (12) is immobilized, having at least one label (8), featuring at least one peroxidase, for labelling the ligand (2), and having luminol (6) which is stabilized with at least one salt, **characterized in that** the inner cavity features at least two compartments (5a, 5b, 5c, 5d), that a sodium-ascorbate-comprising solid (7), in which the label (8) is present in lyophilized form, is deposited in at least one first compartment (5a), and that the salt-stabilized luminol and an ammonium-persulphate-comprising solid substance (10), which releases hydrogen peroxide upon contact with water, are present in at least one second compartment (5b).

5. Apparatus (1) according to Claim 4, **characterized in that** the label (8) features at least one streptavidin-peroxidase complex.

6. Apparatus (1) according to either Claim 4 or 5, **characterized in that** at least one detection antibody (15) which specifically binds the ligand (2) or a functional fragment of such an antibody is present in lyophilized form in the at least one first compartment (5a).

7. Apparatus (1) according to either Claim 5 or 6, **characterized in that** the inner cavity features a third compartment (5c) in which the at least one receptor (12) is immobilized, and that the first compartment (5a) and the second compartment (5b) feature, in each case, an inlet port (9a, 9b) and, in each case, an outlet port (11a, 11b) connected to the third compartment (5c).

8. Apparatus (1) according to any of Claims 4 to 7, **characterized in that** the third compartment (5c) features a fluid outlet (13), and that the at least one receptor (12) is arranged between the first compartment (5a) and/or second compartment (5b) on one side and the fluid outlet (13) on the other side.

## Revendications

1. Nécessaire destiné à la production d'un rayonnement chimiluminescent (16) en fonction de la liaison d'au moins un ligand (2), contenu dans un échantillon à étudier, à au moins un récepteur (12) spécifique de liaison pour le ligand (2), comportant une biopuce, qui comporte un support sur lequel est immobilisé ledit au moins un récepteur (12), comportant au moins un marqueur (8) qui comporte au moins une enzyme peroxydase pour le marquage du ligand (2) et comportant du luminol (6) qui est stabilisé par un sel, **caractérisé en ce que** le nécessaire comporte une substance solide (10) contenant du persulfate d'ammonium, qui libère du peroxyde d'hydrogène lors d'un contact avec l'eau, et **en ce que** le nécessaire comprend un solide (7), contenant de l'ascorbate de sodium, qui contient le marqueur (8) sous forme lyophilisée.

2. Nécessaire selon la revendication 1, **caractérisé en ce que** le marqueur (8) comporte au moins un complexe streptavidine-enzyme peroxydase.

3. Nécessaire selon la revendication 1 ou 2, **caractérisé en ce qu'**il contient sous forme lyophilisée au moins un anticorps de détection (15) spécifique de liaison pour le ligand (2) ou un fragment fonctionnel d'un tel anticorps, et **en ce que** l'anticorps de détection (15) est lié à l'enzyme peroxydase et/ou lors d'un contact avec l'enzyme peroxydase se lie directement à l'enzyme peroxydase ou indirectement à celle-ci.

4. Dispositif (1) destiné à la production d'un rayonnement chimiluminescent (16) en fonction de la liaison d'au moins un ligand (2), contenu dans un échantillon à étudier, à au moins un récepteur (12) spécifique de liaison pour le ligand (2), comportant une cellule à flux (3) comportant une cavité interne, la cavité interne présentant une paroi limitante, sur laquelle est immobilisé ledit au moins un récepteur (12), comportant au moins un marqueur (8) qui comporte au moins une enzyme peroxydase pour le marquage du ligand (2) et comportant du luminol (6) qui est stabilisé par au moins un sel, **caractérisé en ce que** la cavité interne présente au moins deux compartiments (5a, 5b, 5c, 5d), **en ce que** dans au moins un premier compartiment (5a) est déposé un solide (7) contenant de l'ascorbate de sodium, qui contient le marqueur (8) sous forme lyophilisée, et **en ce que** dans au moins un deuxième compartiment (5b) sont contenus le luminol stabilisé par le sel et une substance solide (10) comportant du persulfate d'ammonium, qui libère du peroxyde d'hydrogène lors d'un contact avec l'eau.

5. Dispositif (1) selon la revendication 4, **caractérisé en ce que** le marqueur (8) comporte au moins un complexe streptavidine-enzyme peroxydase.

6. Dispositif (1) selon la revendication 4 ou 5, **caractérisé en ce que** ledit au moins un premier compartiment (5a) contient sous forme lyophilisée au moins un anticorps de détection (15) spécifique de liaison pour le ligand (2) ou un fragment fonctionnel d'un tel anticorps.

7. Dispositif (1) selon l'une quelconque des revendications 5 et 6, **caractérisé en ce que** la cavité interne comporte un troisième compartiment (5c), dans lequel est immobilisé au moins un récepteur (12), et **en ce que** le premier compartiment (5a) et le deuxième compartiment (5b) comportent chacun un orifice d'entrée (9a, 9b) et chacun un orifice de sortie (11a, 11b) relié au troisième compartiment (5c).

8. Dispositif (1) selon l'une quelconque des revendications 4 à 7, **caractérisé en ce que** le troisième compartiment (5c) comporte une sortie de fluide (13), et **en ce que** ledit au moins un récepteur (12) est disposé entre le premier compartiment (5a) et/ou le deuxième compartiment (5b) d'une part et la sortie de fluide (13) d'autre part.
